# EUROPEAN PATENT SPECIFICATION

(11) **EP 4 314 834 B1**
(45) Date of publication and mention of the grant of the patent: **26.02.2025**
(21) Application number: 22714878.0
(22) Date of filing: 22.03.2022
(51) Int. Cl.: G01N 33/68

(54) **DIAGNOSIS OF ALZHEIMER'S DISEASE BY DETECTING AUTO-ANTIBODIES AGAINST Y-BOX BINDING PROTEIN-1 (YB-1)**
DIAGNOSE VON MORBUS ALZHEIMER DURCH NACHWEIS VON AUTOANTIKÖRPERN GEGEN DAS Y-BOX-BINDENDE PROTEIN-1 (YB-1)
DIAGNOSTIC DE LA MALADIE D'ALZHEIMER PAR DÉTECTION D'AUTO-ANTICORPS CONTRE LA PROTÉINE 1 DE LIAISON DE BOÎTE Y (YB-1)

(30) Priority: 24.03.2021 EP 21164708
(43) Date of publication of application: 07.02.2024
(73) Proprietor: CellTrend GmbH, 14943 Luckenwalde (DE)
(72) Inventor: HEIDECKE, Harald, 14169 Berlin (DE); MERTENS, Peter, 39104 Magdeburg (DE)
(74) Representative: Vossius & Partner Patentanwälte Rechtsanwälte mbB
(86) International application number: PCT/EP2022/057501
(87) International publication number: WO 2022/200355

(56) References cited:
- WO-A1-2017/207616
- WO-A2-2006/067792
- US-A1- 2016 331 806
- NOORI AYUSH ET AL: "Systematic review and meta-analysis of human transcriptomics reveals neuroinflammation, deficient energy metabolism, and proteostasis failure across neurodegeneration", NEUROBIOLOGY OF DISEASE, ELSEVIER, AMSTERDAM, NL, vol. 149, 19 December 2020 (2020-12-19), XP086468194, ISSN: 0969-9961, [retrieved on 20201219], DOI: 10.1016/J.NBD.2020.105225

## Description

### FIELD OF THE INVENTION

The present invention is in the field of medicine, in particular to the field diagnostics of Alzheimer's Disease (AD). Furthermore, it relates to methods, means and kits for diagnosis of Alzheimer's Disease in samples of patients.

### BACKGROUND OF THE INVENTION

Alzheimer's disease (AD) is characterized by accumulation of amyloid-β(Aβ) in plaques, tau proteins in neurofibrillary tangles, neurovascular damage, and neuroinflammation. Clinically, patients suffer a gradual decline of cognitive ability, increased prevalence of neuropsychiatric symptoms, and reduced survival. The disease course is heterogeneous at the individual level, and there are very few blood-based biomarkers that can predict clinical outcomes.

Diagnosis of AD is usually based on the person's medical history, history from relatives, and behavioral observations. Imaging techniques and blood-based biomarkers may be used to exclude other diseases. The diagnosis can only be confirmed with very high accuracy post-mortem when brain material is available and can be examined histologically.

Giil et al. (Journal of Alzheimer's Disease 64 (2018) 761-774) found that endogenous antibodies to multiple receptors were associated with neuropsychiatric symptoms and mortality in Alzheimer's Disease.

Y box binding protein 1 (YB-1) (also known as Y-box transcription factor or nuclease-sensitive element-binding protein 1) is a member of the family of DNA- and RNA-binding proteins with a cold shock domain. YB-1 participates in a wide variety of DNA/RNA-dependent events, including DNA reparation, pre-mRNA splicing, mRNA packaging, and regulation of mRNA stability and translation. At the cellular level, multiple activities of YB-1 are manifested with involvement in cell proliferation and differentiation, stress response, and malignant cell transformation (Lyabin et al., Wiley Interdiscip Rev RNA (2014) 5(1):95-110).

Bobkova et al. (PLoS ONE 10(9): e0138867 (2015)) reported that YB-1 suppressed Alzheimer's Disease progression in two animal models. WO 2015/030628 A2 proposed the application of YB-1 protein and fragments thereof for preparing medicinal agents in treating Alzheimer's Disease.

Autoantibodies against YB-1 have been implicated with the diagnosis of cancer, particularly ovarian cancer (WO 2017/207616 A1). WO 2006/067792 A2 discloses a method for diagnosing AD by determining the level of antibodies against aldolase. Noori et al (Neurobiology of disease, 2020) discusses transcriptomics in neuroinflammation.

However, to date meaningful biomarkers for the diagnosis of AD e.g. from blood samples are missing.

### SUMMARY OF THE INVENTION

The present inventors for the first time found that endogenous antibodies against YB-1 (anti-YB-1 auto-antibodies) are associated with Alzheimer's Disease (AD) making them a biomarker for this disease. The data presented herein demonstrate that auto-antibodies directed against YB-1 provide an advantageous tool for the diagnosis of AD.

Hence, the present invention relates to a method for the diagnosis of Alzheimer's Disease (AD) in a subject, comprising the steps of
(i) determining the level of antibodies against Y box binding protein 1 (YB-1) in a sample from a subject to be diagnosed,
(ii) comparing the determined level in the sample to a control level of YB-1 antibodies derived from subjects without AD;
wherein an increased level in the sample from the subject to be diagnosed as compared to the control level is indicative of AD in the subject to be diagnosed.

Particularly, the antibodies against YB-1 may specifically bind to a peptide having a sequence according to SEQ ID NO:2. Hence, in the methods, assays, uses and kits herein, the anti-YB-1 auto-antibodies can advantageously be detected by using peptide immunoassays comprising detecting the binding of the auto-antibodies to a peptide having an epitope in the sequence of SEQ ID NO:2.

The samples in which the anti-YB-1 auto-antibodies are detected are typically blood samples, i.e. whole blood, serum or plasma samples, preferably the sample is plasma or serum. However, the samples may also be cerebrospinal fluid samples (liquor cerebrospinalis; herein in short "liquor").

The method of the invention may be an immunoassay and comprise the steps of
(a) contacting the sample suspected of comprising an YB-1 antibody with YB-1 or an antigenic peptide fragment thereof,
(b) detecting binding of the antibodies to YB-1 or the antigenic peptide fragment thereof.

In particular aspects, the peptide is a peptide consisting of the sequence of SEQ ID NO:2. More in particular, the peptide may comprise 10 to 19, preferably 12 to 19, consecutive residues of SEQ ID NO:2, wherein the peptide is between 10 and 30, preferably 12 to 24, amino residues in length.

The present invention also pertains to the in vitro use of Y-box binding protein 1 (YB-1) or an antigenic peptide fragment thereof for the diagnosis of AD. Further, the invention relates to a kit for diagnosing AD, said kit comprising YB-1 or an antigenic peptide thereof. Again, the antigenic peptide may comprise at least 10 to 19, preferably 12 to 19, consecutive residues of SEQ ID NO:2, and wherein the peptide is between 10 and 30, preferably 12 to 24, amino residues in length.

### DESCRIPTION OF DRAWINGS

Fig. 1 shows a ROC plot for the ELISA assay with peptide 4+5.
Fig. 2 shows the comparison of antibody signal levels of healthy controls with AD patients from the ELISA using peptide 4+5.
Fig. 3 shows a ROC plot for the ELISA assay with peptide 49.
Fig. 4 shows the comparison of antibody signal levels of healthy controls with AD patients from the ELISA using peptide 49.

### DETAILED DESCRIPTION OF THE INVENTION

The present invention is based on the surprising finding of the inventors that in samples of patients with AD increased levels of anti-YB-1 antibodies can be found as compared to subjects without AD ("healthy subjects" or "control subjects"). In other words the inventors have found that patients with AD have higher levels of antibodies against Y-box binding protein-1 (YB-1) in the blood (e.g. determined in serum or plasma) whereas in control groups anti-YB-1 auto-antibodies can be detected at much lower levels.

The present invention is based on the finding that levels of the anti-YB-1 autoimmune-antibodies in subjects have diagnostic properties. The antibodies to be detected in connection with the present invention are therefore autoantibodies, i.e. those produced by immune system of the subject to be diagnosed.

The invention relates to a method for the diagnosis of Alzheimer's Disease (AD) in a subject, comprising the steps of
(i) determining the level of antibodies against Y-box binding protein-1 (YB-1) in a sample from a subject to be diagnosed,
(ii) comparing the determined level in the sample to a control level of YB-1 antibodies derived from subjects without AD;
wherein an increased level in the sample from the subject to be diagnosed as compared to the control level is indicative of AD in the subject to be diagnosed.

The method of the invention works particularly well when detecting auto-antibodies against YB-1 which specifically bind to a peptide having a sequence according to SEQ ID NO:2, i.e. that are specific for an epitope in SEQ ID NO:2.

The skilled person will also understand that the "control" level may be implicated in the used assay for detecting said auto antibodies. The skilled person hence may chose particulars of the assay so that the test is positive for the presence of the antibody in the sample if levels above a certain level is reached and *vice versa* be negative for the presence of said autoantibody if levels are determined that are below the control value.

A level of anti-YB-1 antibodies in the sample of the patient to be diagnosed of more than 1.2 fold as compared to the control level is in certain aspects of the invention indicative of AD in the subject to be diagnosed.

The terms "anti-YB-1 antibody", "YB-1 antibody" and YB-1 auto-antibody" are used synonymously herein and refer to the antibodies present in subjects that specifically bind to YB-1 or an immunogenic fragment thereof. In this context the term "specific binding" refers to antibodies raised against peptides derived from YB-1. Such peptides can comprise additional or less N- or C-terminal amino acids. An antibody is considered to be specific to YB-1 or an immunogenic peptide thereof, if its affinity towards YB-1 is at least 50-fold higher, preferably 100-fold higher, more preferably at least 1000-fold higher than towards other proteins or peptides. It is well known in the art how to determine binding of antibodies to a specific antigen.

The skilled person is aware that for a differential diagnosis of Alzheimer's Disease further parameters to diagnose the subject may be considered in addition, e.g. from imaging methods, patient interviews or further biomarkers. In the context of the present invention the subject to be diagnosed is a human. The subject is preferably a human suspected to have AD.

In the context of the present invention the terms "YB1" or "YBX1" or "YB-1" are interchangeably used herein and refer to "Y-box binding protein-1", "Y-box transcription factor" or "nuclease-sensitive element-binding protein 1", and are also known as BP-8; CSDB; DBPB; CSDA2; NSEP1; NSEP-1; or MDR-NF1. This protein is a protein that in humans is encoded by the YBX1 gene (Entrez Gene ID: 4904). In context with the present invention the subject is preferably a human. YB-1 is a cold shock protein. YB-1 lacks an N-terminal signal peptide motif and therefore its secretion is regulated similar to that of other leaderless proteins. Further to the full-length YB-1 protein which is 324 amino acids in length, a truncated fragment could be detected (Frye et al.; Y-box protein-1 is actively secreted through a non-classical pathway and acts as an extracellular mitogen. EMBO reports 2009, 10(7):783-789).

In the context of the immunoassays of the present invention the "YB-1" may be present in its natural cellular environment and can be used together with the material associated with YB-1 in its natural state as well as in isolated form with respect to its primary, secondary and tertiary structures. The YB-1 is well known to those skilled in the art. The protein or its immunogenic fragment is preferably used in isolated form, i.e. essentially free of other proteins, lipids, carbohydrates or other substances naturally associated with YB-1. "Essentially free of" means that the protein or its immunogenic fragment is at least 75%, preferably at least 85%, more preferably at least 95% and especially preferably at least 99% free of other proteins, lipids, carbohydrates or other substances naturally associated with YB-1.

In connection with the present invention, the naturally occurring protein as well as all modifications, mutants or derivatives of the YB-1 can be used. This includes all naturally present modifications as known to the skilled person. Similarly, a YB-1 produced by means of recombinant techniques, which includes amino acid modifications, such as inversions, deletions, insertions, additions etc. can be used according to the invention provided that this part of the essential function of the YB-1 is present, namely the capability of binding antibodies. The YB-1 being used may also comprise exceptional amino acids and/or modifications of such as alkylation, oxidation, thiol-modification, denaturation, oligomerization and the like. The YB-1 can also be synthesized by chemical means. According to the invention the YB-1 particularly can be a protein and/or peptide or a fusion protein, which in addition to other proteins, peptides or fragments thereof, includes the YB-1 as a whole or in part. Using conventional methods, peptides or polypeptides of the YB-1 which have functionally analogs, analogous properties can be determined by those skilled in the art. For example such polypeptides or peptides have 50-60%, 70% or 80%, preferably 90%, more preferably 95%, and most preferably 98% sequence homology to peptides identified as YB-1, and said homology can be determined, e.g. by means of Smith-Waterman homology search algorithm, using the MPFRCH program (Oxford Molecular), for example.

The term "peptide" or "polypeptide" of a YB-1 used in the present invention comprises also molecules differing from the original sequence by deletion(s), insertion(s), substitution(s) and/or other modifications well known in the prior art and/or comprising a fragment of the original amino acid molecule, with the YB-1 still exhibiting the properties mentioned above. Such a peptide has preferably at least a length of 57 amino acid residues but may also be shorter, e.g. at least 12, 15, 20 or 25 amino acid residues in length. Also included are allele variants and modifications. Methods of producing the above changes in the amino acid sequence are well known to those skilled in the art and have been described in the standard textbooks of molecular biology, e.g. Sambrook et al., "Molecular cloning" (2012) Cold Spring Harbor Laboratory Press, ISBN 978-1-936113-42-2. Those skilled in the art will also be able to determine whether a YB-1, thus, modified still has the properties mentioned above. The amino acid sequence of YB-1 is known. Database entries exist in several well known Databases. When referring to the amino acid sequence of YB-1 any amino acid sequence known is meant, particularly those disclosed in common databases, preferably of human origin. This protein in humans is encoded by the YBX1 gene (Entrez Gene ID: 4904). One preferred sequence of YB-1 are given herein, i.e. SEQ ID NO:1 for full-length YB-1. The YB-1 may be glycosylated *in vivo.* In the present specification all of the above illustrated modifications of the YB-1 will be referred to as "functionally analogous peptides or proteins" or "immunogenic peptides" or "antigenic peptides" in brief.

The antibodies to be detected or determined according to the present invention are directed against YB-1. This means that the antibodies specifically bind YB-1. Specific binding of an antibody normally occurs via binding of a binding site of the antigen. The antibodies of the present invention are those specifically binding to YB-1 or immunogenic fragments thereof. This binding may occur via recognition of sequence or structural epitopes. The skilled person is aware of methods of how determining specific epitopes, e.g. fragments of the antigen YB-1, which are recognized and bound by the antibodies to be determined. Fragments of YB-1 binding to the autoantibodies are called immunogenic or antigenic fragments. An example herein is the peptide of SEQ ID NO:2. Methods for determining fragments of an antigen binding the antibody are described in several publications (e.g. Gershoni, JM; Roitburd-Berman, A; Siman-Tov, DD; Tarnovitski Freund, N; Weiss, Y (2007). "Epitope mapping: The first step in developing epitope-based vaccines". BioDrugs 21 (3): 145-56; Westwood, MR; Hay, FC (2001). Epitope Mapping: a practical approach. Oxford, Oxfordshire: Oxford University Press. ISBN 0-19-963652-4; Flanagan et al. (2011), "Mapping Epitopes with H/D-Ex Mass Spec". Genetic Engineering and Biotechnology news; 31(1); Gaseitsiwe, S.; Valentini, D.; Mahdavifar, S.; Reilly, M.; Ehrnst, A.; Maeurer, M. (2009) "Peptide Microarray-Based Identification of Mycobacterium tuberculosis Epitope Binding to HLA-DRB1*0101, DRB1*1501, and DRB1*0401". Clinical and Vaccine Immunology 17 (1): 168-75; Linnebacher, Michael; Lorenz, Peter; Koy, Cornelia; Jahnke, Annika; Born, Nadine; Steinbeck, Felix; Wollbold, Johannes; Latzkow, Tobias et al. (2012). "Clonality characterization of natural epitope-specific antibodies against the tumor-related antigen topoisomerase Ila by peptide chip and proteome analysis: A pilot study with colorectal carcinoma patient samples" Analytical and Bioanalytical Chemistry 403 (1): 227-38; Cragg, M. S. (2011). "CD20 antibodies: Doing the time warp". Blood 118 (2): 219-20; Banik, Soma S. R.; Doranz, Benjamin J. (2010). "Mapping Complex Antibody Epitopes". Genetic Engineering and Biotechnology News 3 (2): 25-8; and Paes, Cheryl; Ingalls, Jada; Kampani, Karan; Sulli, Chidananda; Kakkar, Esha; Murray, Meredith; Kotelnikov, Valery; Greene, Tiffani A. et al. (2009). "Atomic-Level Mapping of Antibody Epitopes on a GPCR". Journal of the American Chemical Society 131 (20): 6952-4). In context with the present invention anti-YB-1 antibodies are understood as any immunoglobulin specifically recognizing/binding to YB-1. The antibody to be detected binds any of YB-1, preferably to a sequence comprising or consisting of SEQ ID NO:1 or SEQ ID NO:2.

In the context of the present invention the anti-YB-1 antibody may an IgA-antibody, IgM-antibody and IgG-antibody, e.g. IgG1, IgG2, IgG3 and IgG4.

The control levels as disclosed herein refer to control levels of YB-1 antibodies. It will be readily understood by the skilled person that the control levels from subjects having the desired disease or response as defined in the methods and to which the determined levels are compared to, are not necessarily determined in parallel but may be represented by previously determined levels. Nevertheless, control levels may be determined in parallel. The skilled person with the disclosure of the present invention and his knowledge is able to determine such levels, as outlined herein. Hence, the control levels of the present invention may be previously defined thresholds or cut-off values. Preferred thresholds are disclosed herein. Furthermore, it will be acknowledged by the skilled person that control levels are, like the levels to be determined in the subject to be diagnosed, determined in samples of the subjects not having AD, i.e. in this context healthy subjects. Preferably, the sample is the same kind of sample as the sample of the person to be diagnosed or to be treated, e.g. when the sample of the latter is serum, the control levels are preferably derived from serum samples of the control subjects.

As outlined herein, the levels of YB-1 antibodies in samples of the patient to be diagnosed are compared with the control groups as defined herein. However, in certain aspects the levels are compared to fixed values, i.e. thresholds under or over which a certain diagnosis, or prognosis of response is given. To this end, unit-standards may be applied (e.g. see WO 2017/207616 A1). Such standard for the YB-1 antibody can e.g. be derived from a serum sample from a systemic sclerosis patient. Systemic sclerosis patients are known to have high levels of autoimmune antibodies in general. However, it will be acknowledged by the skilled person that also other samples may be taken to set a different standard, e.g. samples of healthy subjects. Nevertheless, the principle of generating a standard (units) is the same in any case and are exemplified herein using serum samples of systemic sclerosis patients.

"Equal" level in context with the present invention means that the levels differ by not more than ± 10 %, preferably by not more than ± 5 %, more preferably by not more than ± 2 %. Thus, "decreased" or "increased" level in the context of the present invention mean that the levels differ by more than 10 %, preferably by more than 15 %, preferably more than 20 %.

Herein, the sample of the subject to be diagnosed in which the level of anti-YB-1 antibodies is to be determined is preferably a bodily fluid such as whole blood, liquor or lymph or fractions of blood such as serum or plasma. Preferably in the context of the present invention the sample is plasma or serum or liquor.

Where appropriate, the sample may need to be homogenized, or extracted with a solvent prior to use in the present invention in order to obtain a liquid sample. A liquid sample hereby may be a solution or suspension. Liquid samples may be subjected to one or more pre-treatments prior to use in the present invention. Such pre-treatments include, but are not limited to dilution, filtration, centrifugation, concentration, sedimentation, precipitation, and dialysis. Pre-treatments may also include the addition of chemical or biochemical substances to the solution, such as acids, bases, buffers, salts, solvents, reactive dyes, detergents, emulsifiers, chelators.

"Plasma" in the context of the present invention is the virtually cell-free supernatant of blood containing anticoagulant obtained after centrifugation. Exemplary anticoagulants include calcium ion binding compounds such as EDTA or citrate and thrombin inhibitors such as heparinates or hirudin. Cell-free plasma can be obtained by centrifugation of the anticoagulated blood (e.g. citrated, EDTA or heparinized blood) for at least 15 minutes at 2000 to 3000 g.

"Serum" is the liquid fraction of whole blood that is collected after the blood is allowed to clot. When coagulated blood (clotted blood) is centrifuged serum can be obtained as supernatant. It does not contain fibrinogen, although some clotting factors remain.

"Liquor" is cerebrospinal fluid that can be obtained by lumbar puncture.

In the methods of the present invention, the anti-YB-1 antibody is preferably detected in an immunoassay. Suitable immunoassays may be selected from the group of immunoprecipitation, enzyme immunoassay (EIA)), enzyme-linked immunosorbentassays (ELISA), radioimmunoassay (RIA), fluorescent immunoassay, a chemiluminescent assay, an agglutination assay, nephelometric assay, turbidimetric assay, a Western Blot, a competitive immunoassay, a noncompetitive immunoassay, a homogeneous immunoassay a heterogeneous immunoassay, a bioassay and a reporter assay such as a luciferase assay or luminex. Preferably herein the immunoassay is an enzyme linked immunosorbent assay (ELISA). Luminex assays are also preferred herein.

The immunoassays can be homogenous or heterogeneous assays, competitive and noncompetitive assays. The assay may be in the form of a sandwich assay, which is a noncompetitive immunoassay, wherein the anti-YB-1 antibody (i.e. the "analyte") to be detected and/or quantified is allowed to bind to an immobilized YB-1 protein or immunogenic peptide fragment thereof and to a secondary antibody. The YB-1 or fragment thereof (i.e. a peptide), may e.g., be bound to a solid phase, e.g. a bead, a surface of a well or other container, a chip or a strip, and the secondary antibody is an antibody which is labeled, e.g. with a dye, with a radioisotope, or a reactive or catalytically active moiety such as a peroxidase, e.g. horseradish peroxidase. The amount of labeled antibody bound to the analyte is then measured by an appropriate method. The general composition and procedures involved with "sandwich assays" are well-established and known to the skilled person (The Immunoassay Handbook, Ed. David Wild, Elsevier LTD, Oxford; 3rd ed. (May 2005), ISBN-13: 978-0080445267*;* Hultschig C et al., Curr Opin Chem Biol. 2006 Feb;10(1):4-10. PMID: 16376134. Sandwich immunoassays can for example be designed as one-step assays or as two-step assays.

The detectable label may for example be based on fluorescence or chemiluminescence. The labelling system comprises rare earth cryptates or rare earth chelates in combination with a fluorescence dye or chemiluminescence dye, in particular a dye of the cyanine type. In the context of the present invention, fluorescence based assays comprise the use of dyes, which may for instance be selected from the group comprising FAM (5-or 6-carboxyfluorescein), VIC, NED, Fluorescein, Fluoresceinisothiocyanate (FITC), IRD-700/800, Cyanine dyes, such as CY3, CY5, CY3.5, CY5.5, Cy7, Xanthen, 6-Carboxy-2',4',7',4,7-hexachlorofluorescein (HEX), TET, 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like.

In the context of the present invention, chemiluminescence based assays comprise the use of dyes, based on the physical principles described for chemiluminescent materials in Kirk-Othmer, Encyclopedia of chemical technology, 4th ed., executive editor, J. I. Kroschwitz; editor, M. Howe-Grant, John Wiley & Sons, 1993, vol.15, p. 518-562*,* including citations on pages 551-562. Preferred chemiluminescent dyes are acridiniumesters.

The "sensitivity" of an assay relates to the proportion of actual positives which are correctly identified as such, i.e. the ability to identify positive results (true positives positive results / number of positives). Hence, the lower the concentrations of the analyte that can be detected with an assay, the more sensitive the immunoassay is. The "specificity" of an assay relates to the proportion of negatives which are correctly identified as such, i.e. the ability to identify negative results (true negatives / negative results). For an antibody the "specificity" is defined as the ability of an individual antigen binding site to react with only one antigenic epitope. The binding behaviour of an antibody can also be characterized in terms of its "affinity" and its "avidity". The "affinity" of an antibody is a measure for the strength of the reaction between a single antigenic epitope and a single antigen binding site. The "avidity" of an antibody is a measure for the overall strength of binding between an antigen with many epitopes and multivalent antibodies.

An "immunogenic peptide" or "antigenic peptide" as used herein is a portion of a YB-1 protein that is recognized (i.e., specifically bound) by the anti-YB-1 antibody such as the peptide according to SEQ ID NO:2. Such immunogenic peptides generally comprise at least 5 amino acid residues, more preferably at least 10, and still more preferably at least 20 amino acid residues of YB-1. However, they may also comprise at least 30, 40, 50, 60, 70, 80, 90, 100, 110, 120, 130, 140 or 150 amino acid residues.

For the purposes of the immunoassays and diagnostic methods of the invention, YB-1 can be produced by expression in cells, preferably eukaryotic cells or in cell free, preferably eukaryotic cell free systems. Polypeptide and peptide fragments of YB-1 can also be produced synthetically or by enzymatic digestions. Hence, in the assays and methods of the invention YB-1 may be present in its natural cellular environment and can be used together with the material associated with the protein in its natural state as well as in isolated form. Suitable expression systems include prokaryotic cells (e.g. E. coli) or eukaryotic cells (e.g. mammalian cells such as Chinese hamster ovary (CHO) cells) overexpressing the human YB-1. Cell extracts or purified protein can be used to detect anti-YB-1 antibodies. Based on the weight of the whole protein or its immunogenic fragment in the preparation (e.g. the "extract") to be used according to the invention, the isolated protein should account for at least 0.5%, preferably at least 5% more preferably at least 25%, and particular preferred at least 50%. The protein is preferably used in isolated form, i.e. essentially free of other proteins, lipids, carbohydrates or other substances naturally associated with the YB-1. "Essentially free of" means that the protein is at least 75%, preferably at least 85%, more preferably at least 95% and especially preferably at least 99% free of other proteins, lipids, carbohydrates or other substances naturally associated with the protein.

In some aspects of the present invention, the method comprises the steps of
(a) contacting the sample suspected of comprising an YB-1 antibody with YB-1 or an antigenic peptide fragment thereof,
(b) detecting binding of the antibodies to YB-1 or the antigenic peptide fragment thereof.

The antigenic peptide may advantageously be a peptide according to the sequence of SEQ ID NO:2 (e.g. consisting of SEQ ID NO:2 or comprising at least 10 to 19 consecutive residues of SEQ ID NO:2, wherein the peptide is between 10 and 30 amino residues in length). Hence in a preferred aspect of the method of the invention, the peptide comprises 10 to 19, preferably 12 to 19, consecutive residues of SEQ ID NO:2, wherein the peptide is between 10 and 30, preferably 12 to 24, amino residues in length.

E.g. that may mean that the method comprises the steps of
(a) contacting the sample with Y-box binding protein-1 (YB-1) or an antigenic peptide fragment thereof under conditions allowing for the formation of a complex between anti-YB-1 antibodies with YB-1 or a peptide fragment thereof,
(b) detecting the complex.

In certain aspects the method comprises the steps of
(a) contacting the sample with a peptide comprising at least 10 to 19, preferably 12 to 19, consecutive residues of SEQ ID NO:2 under conditions allowing for the formation of a complex between the anti-YB-1 antibodies and the peptide, wherein the peptide is between 10 and 30, preferably 12 to 24, amino residues in length,
(b) detecting the complex.

In other words, the method of the invention may comprise the steps of
(a) contacting the sample with a peptide comprising 10 to 19, preferably 12 to 19, consecutive residues of SEQ ID NO:2, wherein the peptide is between 10 and 30, preferably 12 to 24, amino residues in length,
(b) detecting binding of the antibodies to the peptide.

The present invention also relates to a method for determining the level of anti-YB-1 auto-antibodies in a sample from a subject, comprising the step of
(a) contacting the sample with a peptide comprising 10 to 19, preferably 12 to 19, consecutive residues of SEQ ID NO:2, wherein the peptide is between 10 and 30, preferably 12 to 24, amino residues in length,
(b) detecting binding of the antibodies to the peptide.

This method for determining the level of anti-YB-1 auto-antibodies in a sample from a subject may further comprise the comparison of the anti-YB-1 auto-antibody level in the sample of the subject with a pre-determined threshold level determined on the basis of the comparison of such levels in healthy subjects and in patients suffering from Alzheimer's Disease.

Herein, the full-length YB-1 (e.g. see WO 2017/207616 A1) or the antigenic peptide fragment (such as the preferred peptide based on or derived from SEQ ID NO:2 mentioned herein above) thereof may preferably be immobilized on a surface. In a preferred aspect, the YB-1 or the antigenic peptide fragment thereof is immobilized directly on a surface. "Direct" immobilization in context with the present invention relates to an immobilization without using tags fused to the YB-1, e.g. without YB-1 being fused to a GST-Tag. A direct immobilization to the surface means that the protein/peptide is not fused to a tag. Such tag may interfere with the conformation of the protein that could cause the autoantibodies to be detected no longer binding to the protein. Hence, conformation stabilizing means are preferred in context with the methods according to the invention. Preferably the YB-1 is immobilized directly on a surface or stabilized by a buffer covering the surface, the buffer comprising Calcium chloride, further preferred the YB-1 is immobilized directly on a surface and stabilized by a buffer covering the surface, the buffer comprising Calcium chloride. The Calcium chloride concentration of the buffer may vary, however it shall stabilize the protein to allow proper binding of antibodies to the YB-1. In a preferred aspect the Calcium chloride concentration is from 0.1 mM to 10 mM, further preferred from 0.5 to 5 mM, particularly preferred 1 mM. The calcium chloride may in a preferred aspect be added to any buffer of the methods of the present invention that come into contact with the YB-1 protein, e.g. also washing and/or detection buffers.

The complex may for example be detected using a secondary antibody against the Fc portion of the anti-YB-1 antibody. When the anti-YB-1 antibody is an IgG-antibody, the secondary antibody may be an anti-IgG antibody. Hence, the anti-YB-1 antibody to be detected may be an IgG-antibody and the secondary antibody is an anti-IgG antibody, particularly preferred the subject is a human and the second antibody is an anti-human-IgG antibody. The skilled person will understand that it is possible to detect total IgG, i.e. the method does not distinguish between the subtypes of IgG antibodies. Hence, in one aspect the secondary antibody is an anti-human-total IgG antibody. Nevertheless, in some aspects it may be preferred that the subtypes are differentially detected. Hence, in a particular aspect, the subject is a human and
(i) the anti-YB-1 antibody is an IgG1-antibody and the secondary antibody is an anti-human-IgG1 antibody; or
(ii) the anti-YB-1 antibody is an IgG2-antibody and the secondary antibody is an anti-human-IgG2 antibody; or
(iii) the anti-YB-1 antibody is an IgG3-antibody and the secondary antibody is an anti-human-IgG3 antibody; or
(iv) the anti-YB-1 antibody is an IgG4-antibody and the secondary antibody is an anti-human-IgG4 antibody.

The secondary antibody may for example be labeled with a detectable marker, e.g. a peroxidase.

The invention also relates to the use of YB-1 or an antigenic peptide fragment thereof, preferably as set out herein above (e.g. the antigenic peptide comprises at least 10 to 19, preferably 12 to 19, consecutive residues of SEQ ID NO:2, and wherein the peptide is between 10 and 30, preferably 12 to 24, amino residues in length), for the diagnosis of Alzheimer's Disease. Hence, the antigenic peptide as such may be used for detection of anti-YB-1 antibodies in a sample of a subject to be diagnosed for AD.

In the context of the present invention, the levels of the anti-YB-1 antibodies may be analyzed in a number of fashions well known to a person skilled in the art. For example, each assay result obtained may be compared to a "normal" (control) value, or a value indicating a particular disease or outcome. A particular diagnosis/prognosis may depend upon the comparison of each assay result to such a value, which may be referred to as a diagnostic or prognostic "threshold". In certain aspects, assays for one or more diagnostic or prognostic indicators are correlated to a condition or disease by merely the presence or absence of the indicator(s) in the assay. For example, an assay can be designed so that a positive signal only occurs above a particular threshold concentration of interest, and below which concentration the assay provides no signal above background.

The sensitivity and specificity of a diagnostic and/or prognostic test depends on more than just the analytical "quality" of the test, they also depend on the definition of what constitutes an abnormal result. In practice, Receiver Operating Characteristic curves (ROC curves), are typically calculated by plotting the value of a variable versus its relative frequency in "normal" (i.e. apparently healthy individuals not having AD) and "disease" (AD) populations. For any particular marker, a distribution of marker levels for subjects with and without the disease will likely overlap. Under such conditions, a test does not absolutely distinguish normal from disease with 100% accuracy, and the area of overlap indicates where the test cannot distinguish normal from disease. A threshold is selected, above which the test is considered to be abnormal and below which the test is considered to be normal (healthy). The area under the ROC curve is a measure of the probability that the perceived measurement will allow correct identification of a condition. ROC curves can be used even when test results do not necessarily give an accurate number. As long as one can rank results, one can create a ROC curve. For example, results of a test on "disease" samples might be ranked according to degree (e.g. 1=low, 2=normal, and 3=high). This ranking can be correlated to results in the "normal" population, and a ROC curve created. These methods are well known in the art. See, e.g., Hanley et al. 1982. Radiology 143: 29-36*.* Preferably, a threshold is selected to provide a ROC curve area of greater than about 0.5, more preferably greater than about 0.7, still more preferably greater than about 0.8, even more preferably greater than about 0.85, and most preferably greater than about 0.9. The term "about" in this context refers to +/- 5% of a given measurement.

The horizontal axis of the ROC curve represents (1-specificity), which increases with the rate of false positives. The vertical axis of the curve represents sensitivity, which increases with the rate of true positives. Thus, for a particular cut-off selected, the value of (1-specificity) may be determined, and a corresponding sensitivity may be obtained. The area under the ROC curve is a measure of the probability that the measured marker level will allow correct identification of a disease or condition. Thus, the area under the ROC curve can be used to determine the effectiveness of the test.

A positive likelihood ratio, negative likelihood ratio, odds ratio, or hazard ratio may be used as a measure of a test's ability to predict risk or diagnose a disease. In the case of a positive likelihood ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group.

In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group.

In the case of a hazard ratio, a value of 1 indicates that the relative risk of an endpoint (e.g., death) is equal in both the "diseased" and "control" groups; a value greater than 1 indicates that the risk is greater in the diseased group; and a value less than 1 indicates that the risk is greater in the control group.

The skilled artisan will understand that associating a diagnostic or prognostic indicator, with a diagnosis or with a prognostic risk of a future clinical outcome is a statistical analysis. For example, a marker level of higher than X may signal that a patient is more likely to suffer from AD than patients with a level lower than or equal to X, as determined by a level of statistical significance. Additionally, a change in marker concentration from baseline levels may be reflective of patient prognosis, and the degree of change in marker level may be related to the severity of adverse events. Statistical significance is often determined by comparing two or more populations, and determining a confidence interval and/or a p value. See, *e.g.,* Dowdy and Wearden, Statistics for Research, John Wiley & Sons, New York, 1983*.* Preferred confidence intervals of the invention are 90%, 95%, 97.5%, 98%, 99%, 99.5%, 99.9% and 99.99%, while preferred p values are 0.1, 0.05, 0.025, 0.02, 0.01, 0.005, 0.001, and 0.0001.

Suitable threshold levels can be determined by grouping a reference population of patients according to their level of YB-1-antibodies into certain quantiles, e.g. quartiles, quintiles or even according to suitable percentiles. Kaplan-Meier estimators may be used for the assessment of a patient.

The invention also pertains to a research and/or diagnostic kit for the diagnosis of Alzheimer's Disease, wherein the kit comprises YB-1 or an antigenic peptide fragment thereof. The kit may further comprise an antibody directed to the Fc portion of the anti-YB-1 antibody to be detected, i.e. an anti-human IgG antibody.

Such kits can comprise a carrier, package or container that is compartmentalized to receive one or more containers such as vials, tubes, and the like, each of the container(s) comprising one of the separate elements to be used in the method. The kit of the invention will typically comprise the container described above and one or more other containers comprising materials desirable from a commercial and user standpoint, including buffers, diluents, filters, needles, syringes, and package inserts with instructions for use. In addition, a label can be provided on the container to indicate that the composition is used for a specific therapeutic or non-therapeutic application, and can also indicate directions for either *in vivo* or *in vitro* use, such as those described herein. Directions and or other information can also be included on an insert which is included with the kit.

Said kit typically comprises the YB-1 protein or an antigenic peptide thereof (as defined herein above) and means to detect antibodies binding to said YB-1 or antigenic peptide thereof. The kit is preferably designed to detect autoimmune antibodies in samples of subject and hence comprises means to detect such antibodies, particularly antibodies binding to said YB-1 or peptide thereof. Such means are outlined herein above, e.g. for immunoassays, and applying likewise to the kit. The aspects set out for the immunoassays, proteins and methods apply also to the kit of the invention. The kits of the present invention are meant for the detection of autoimmune antibodies in samples, e.g. blood samples, like serum or plasma. Hence, in one aspect they comprise means for the preparation of blood, e.g. for gaining serum thereof. The kit may also comprise means for detecting antibodies, e.g. secondary antibodies, as outlined herein above. Furthermore, the kit may comprise control composition and/or standards. The control composition preferably comprises YB-1 antibodies as positive control. Furthermore, the kit may comprise one or a plurality of standard compositions. A standard composition comprises YB-1 antibodies at a defined concentration. As outlined herein, determination of concentration of autoimmune-antibodies may be performed using standard curves. These curves set out which concentration of antibodies in a sample or solution corresponds to what read-out value of the assay used, e.g. optical density or proportion of optical density at different wavelengths (e.g. 450nm/620nm). To this end the kits of the present invention may comprise one or more standard compositions having a defined concentration of YB-1 antibodies, preferably of the kind to be detected in the method.

It will be readily understood that the aspects outlined above shall apply to the invention as a whole and not be limited to a specific method, unless stated otherwise. The invention is further illustrated by the following non-limiting examples and figures.

### SEQUENCES

**SEQ ID NO: 1:**
   Amino acid sequence of YB-1 [SEQ ID NO:1]: 
1 MSSEAETQQP PAAPPAAPAL SAADTKPGTT GSGAGSGGPG GLTSAAPAGG

51 DKKVIATKVL GTVKWFNVRN GYGFINRNDT KEDVFVHQTA IKKNNPRKYL

101 RSVGDGETVE FDVVEGEKGA EAANVTGPGG VPVQGSKYAA DRNHYRRYPR

151 RRGPPRNYQQ NYQNSESGEK NEGSESAPEG QAQQRRPYRR RRFPPYYMRR

201 PYGRRPQYSN PPVQGEVMEG ADNQGAGEQG RPVRQNMYRG YRPRFRRGPP

251 RQRQPREDGN EEDKENQGDE TQGQQPPQRR YRRNFNYRRR RPENPKPQDG

300 KETKAADPPA ENSSAPEAEQ GGAE
**SEQ ID NO: 2:**
   Amino acid sequence of peptide 4+5 [SEQ ID NO:2] (residues 13-31 of SEQ ID NO:1):
   1 APPAAPALSA ADTKPGTTG
**SEQ ID NO: 3:**
   Amino acid sequence of peptide 49 [SEQ ID NO:3] (residues 193-207 of SEQ ID NO:1):
   1 FPPYYMRRPY GRRPQ

### EXAMPLES

### Example 1:

YB-1 autoantibody levels in serum samples were measured using two different sandwich ELISA kits (CellTrend GmbH Luckenwalde, Germany). For the first kit the microtiter 96-well polystyrene plates were directly coated with a peptide according to SEQ ID NO:2 ("peptide 4+5" a.k.a. "peptide 45"). For the second kit the microtiter 96-well polystyrene plates were directly coated with a peptide according to SEQ ID NO:3 ("peptide 49").

To maintain the conformational epitopes of the protein/fragment, 1 mM Calcium chloride was added to every buffer. Duplicate samples of a 1: 100 serum dilution were incubated at 4°C for 2 hours. After washing steps, plates were incubated for 60 minutes with a 1:20.000 dilution of horseradish-peroxidase-labeled goat anti-human IgG (Jackson, USA) used for detection. In order to obtain a standard curve, plates were incubated with test sera from an anti-YB-1 autoantibody positive index patient suffering from systemic sclerosis. The ELISA was validated according to the FDA's "Guidance for industry: Bioanalytical method validation".

YB-1-autoantibodies are not commercially available; a serum sample from a patient with a systemic sclerosis is used for the standard curve. A 1:200 dilution of the serum sample is defined as 40 units/ml YB-1-antibodies. To set a standard for the concentrations of the autoimmune antibodies, a standard curve was generated. In detail, a serum sample of systemic sclerosis serum sample was diluted (a) 1: 200 for standard point 40 units/ml, (b) 1: 400 for standard point 20 units/ml, (c) 1: 800 for standard point 10 units/ml, (d) 1: 1600 for standard point 5 units/ml, (e) 1: 3200 for standard point 2.5 units/ml and (f) 1: 6400 for standard point 1.25 units/ml. Then the optical density was determined using the kit and method of above. Each standard point was performed in duplicates.

### Example 2:

YB-1 antibody levels in serum samples from 9 healthy donors ("Control") and 5 patients with Alzheimer's Disease were measured using the kits and method of example 1 (YB-1-AB-ELISAs). The levels were determined in OD (optical density).

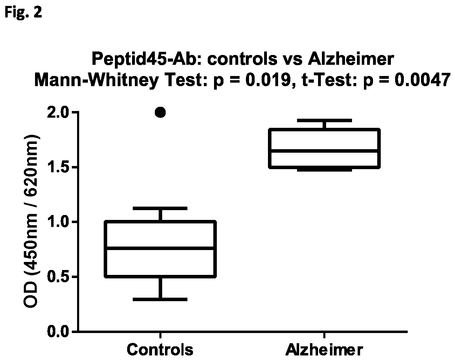

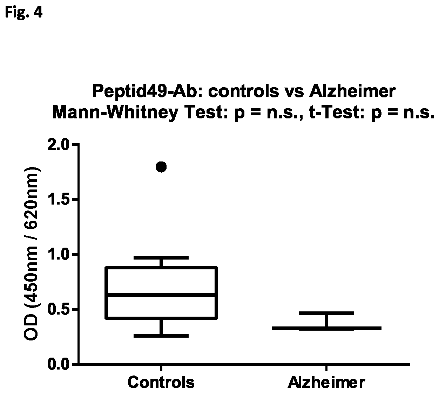

Figures 2 and 4 show the anti-YB-1 antibody levels as determined in serum samples of healthy donors compared to AD patients for the two peptide ELISAs.

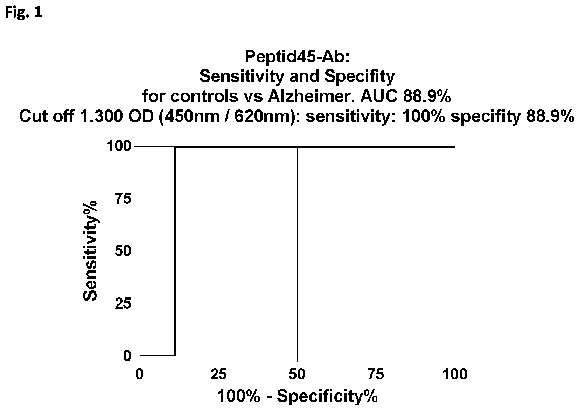

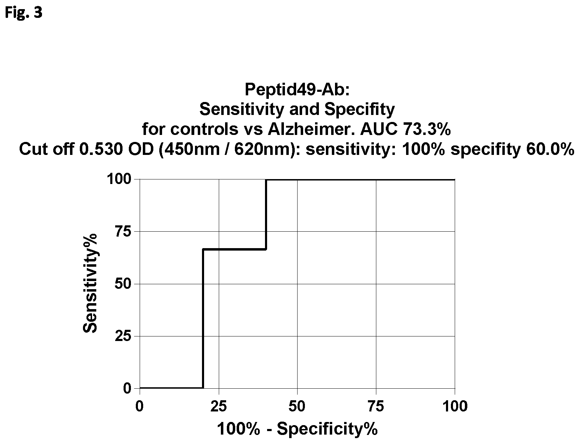

ROC-analyses were performed. The results are shown in Fig. 1 and Fig. 3 for the two YB-1-AB peptide ELISAs. The results clearly show that the use of the two YB-1-AB-ELISAs allows for a good detection of anti-YB-1 antibodies and delivers a diagnostic assays with high sensitivity and specificity.

## Claims

1. A method for the diagnosis of Alzheimer's Disease (AD) in a subject, comprising the steps of
(i) determining the level of antibodies against Y-box binding protein-1 (YB-1) in a sample from a subject to be diagnosed,
(ii) comparing the determined level in the sample to a control level of YB-1 antibodies derived from subjects without AD;
wherein an increased level in the sample from the subject to be diagnosed as compared to the control level is indicative of AD in the subject to be diagnosed.

2. The method of claim 1, wherein the antibodies against YB-1 specifically bind to a peptide having a sequence according to SEQ ID NO:2.

3. The method of claim 1 or 2, wherein a level of anti-YB-1 antibodies in the sample of the patient to be diagnosed of more than 1.2 fold as compared to the control level is indicative of AD in the subject to be diagnosed.

4. The method according to any one of claims 1 to 3, wherein the anti-YB-1 antibodies are detected in an immunoassay.

5. The method according to claim 4, wherein the immunoassay is selected from the group of immunoprecipitation, enzyme immunoassay (EIA), radioimmunoassay (RIA), enzyme-linked immunosorbent assay (ELISA), fluorescent immunoassay, a chemiluminescent assay, an agglutination assay, nephelometric assay, turbidimetric assay, a Western Blot, a competitive immunoassay, a noncompetitive immunoassay, a homogeneous immunoassay a heterogeneous immunoassay, a bioassay and a reporter assay such as a luciferase assay or luminex.

6. The method according to any one of the preceding claims, wherein the sample is plasma or serum.

7. The method of any of the preceding claims, comprising the steps of
(a) contacting the sample suspected of comprising an YB-1 antibody with YB-1 or an antigenic peptide fragment thereof,
(b) detecting binding of the antibodies to YB-1 or the antigenic peptide fragment thereof.

8. The method of claim 7, wherein the peptide is a peptide consisting of the sequence of SEQ ID NO:2.

9. The method according to claim 7, wherein the peptide comprises 10 to 19, preferably 12 to 19, consecutive residues of SEQ ID NO:2, wherein the peptide is between 10 and 30, preferably 12 to 24, amino residues in length.

10. The method of claims 7 to 9, wherein the peptide is immobilized on a surface.

11. The method according to any one of claims 7 to 10, wherein binding is detected using a secondary antibody against the Fc portion of the anti-YB-1 antibody.

12. The method according to claim 11, wherein the anti-YB-1 antibody is an IgG-antibody and the secondary antibody is an anti-IgG antibody.

13. The method according to claims 11 or 12, wherein the secondary antibody is labeled with a detectable marker.

14. In vitro use of Y-box binding protein-1 (YB-1) or an antigenic peptide fragment thereof for the diagnosis of AD.

15. The use of claim 14, wherein the antigenic peptide comprises at least 10 to 19, preferably 12 to 19, consecutive residues of SEQ ID NO:2, and wherein the peptide is between 10 and 30, preferably 12 to 24, amino residues in length.

## Patentansprüche

1. Verfahren zur Diagnose von Alzheimer Krankheit (AD) in einem Individuum, umfassend die Schritte:
(i) Bestimmen des Spiegels von Antikörpern gegen Y-Box-bindendes Protein-1 (YB-1) in einer Probe von einem Individuum, für das eine Diagnose zu stellen ist,
(ii) Vergleichen des bestimmten Spiegels in der Probe mit einem Kontrollspiegel von YB-1-Antikörpern, die von Individuen ohne AD stammen;
wobei ein erhöhter Spiegel in der Probe des Individuums, für das eine Diagnose zu stellen ist, im Vergleich zu dem Kontrollspiegel ein Hinweis auf AD bei dem Individuum ist, für das eine Diagnose zu stellen ist.

2. Verfahren nach Anspruch 1, wobei die Antikörper gegen YB-1 spezifisch an ein Peptid mit der Sequenz gemäß SEQ ID NO:2 binden.

3. Verfahren nach Anspruch 1 oder 2, wobei ein Spiegel von anti-YB-1-Antikörpern in der Probe des Individuums, für das eine Diagnose zu stellen ist, von mehr als das 1,2-Fache im Vergleich zum Kontrollspiegel ein Hinweis auf AD bei dem Individuum ist, für das eine Diagnose zu stellen ist.

4. Verfahren nach einem der Ansprüche 1 bis 3, wobei die anti-YB-1-Antikörper in einem Immunoassay nachgewiesen werden.

5. Verfahren nach Anspruch 4, wobei der Immunoassay ausgewählt ist aus der Gruppe von Immunpräzipitation, Enzym-Immunoassay (EIA), Radio-Immunoassay (RIA), Enzyme-linked Immunosorbent Assay (ELISA), Fluoreszenz-Immunoassay, einem Chemilumineszenz-Assay, einem Agglutinations-Assay, Nephelometrie-Assay, Turbidimetrie-Assay, einem Western Blot, einem kompetitiven Immunoassay, einem nicht-kompetitiven Immunoassay, einem homogenen Immunoassay, einem heterogenen Immunoassay, einem Bioassay und einem Reporter-Assay wie einem Luciferase-Assay oder Luminex.

6. Verfahren nach einem der vorangehenden Ansprüche, wobei die Probe Plasma oder Serum ist.

7. Verfahren nach einem der vorangehenden Ansprüche, umfassend die Schritte:
(a) Inkontaktbringen der Probe, von der vermutet wird, einen YB-1-Antikörper zu umfassen, mit YB-1 oder einem antigenen Peptidfragment davon,
(b) Nachweisen der Bindung der Antikörper an YB-1 oder das antigene Peptidfragment davon.

8. Verfahren nach Anspruch 7, wobei das Peptid ein Peptid ist, das aus der Sequenz von SEQ ID NO:2 besteht.

9. Verfahren nach Anspruch 7, wobei das Peptid 10 bis 19, bevorzugt 12 bis 19, aufeinanderfolgende Reste von SEQ ID NO:2 umfasst, wobei das Peptid eine Länge zwischen 10 und 30, bevorzugt 12 bis 24, Aminoreste aufweist.

10. Verfahren nach den Ansprüchen 7 bis 9, wobei das Peptid auf einer Oberfläche immobilisiert ist.

11. Verfahren nach einem der Ansprüche 7 bis 10, wobei die Bindung unter Verwendung eines sekundären Antikörpers gegen den Fc-Teil des anti-YB-1-Antikörpers nachgewiesen wird.

12. Verfahren nach Anspruch 11, wobei der anti-YB-1-Antikörper ein IgG-Antikörper und der sekundäre Antikörper ein anti-IgG-Antikörper ist.

13. Verfahren nach Anspruch 11 oder 12, wobei der sekundäre Antikörper mit einem nachweisbaren Marker markiert ist.

14. *In* vitro-Verwendung von Y-Box-bindendem Protein-1 (YB-1) oder eines antigenen Peptidfragments davon für die Diagnose von AD.

15. Verwendung nach Anspruch 14, wobei das antigene Peptid mindestens 10 bis 19, bevorzugt 12 bis 19, aufeinanderfolgende Reste von SEQ ID NO:2 umfasst, und wobei das Peptid eine Länge zwischen 10 und 30, bevorzugt 12 bis 24, Aminoreste aufweist.

## Revendications

1. Procédé de diagnostic de la maladie d'Alzheimer (MA) chez un sujet, comprenant les étapes de
(i) détermination du niveau d'anticorps contre la protéine-1 de liaison à la boîte Y (YB-1) dans un échantillon provenant d'un sujet chez lequel un diagnostic est à établir,
(ii) comparaison du niveau déterminé dans l'échantillon à un niveau témoin d'anticorps YB-1 dérivés de sujets sans MA ;
dans lequel un niveau accru dans l'échantillon provenant du sujet chez lequel un diagnostic est à établir par rapport au niveau témoin indique une MA chez le sujet chez lequel un diagnostic est à établir.

2. Procédé selon la revendication 1, dans lequel les anticorps contre YB-1 se lient spécifiquement à un peptide ayant une séquence selon SEQ ID NO : 2.

3. Procédé selon la revendication 1 ou 2, dans lequel un niveau d'anticorps anti-YB-1 dans l'échantillon du patient chez lequel un diagnostic est à établir supérieur à 1,2 fois par rapport au niveau témoin indique une MA chez le sujet chez lequel un diagnostic est à établir.

4. Procédé selon l'une quelconque des revendications 1 à 3, dans lequel les anticorps anti-YB-1 sont détectés dans un immuno-essai.

5. Procédé selon la revendication 4,
dans lequel l'immuno-essai est choisi dans le groupe de l'immunoprécipitation, l'immuno-essai enzymatique (EIA), le radio immuno-essai (RIA), l'essai d'immuno-absorption enzymatique (ELISA), l'immuno-essai par fluorescence, un essai par chimiluminescence, un essai d'agglutination, l'essai néphélométrique, l'essai turbidimétrique, un Western Blot, un essai immunologique par compétition, un immuno-essai non compétitif, un immuno-essai homogène, un immuno-essai hétérogène, un bio-essai et un essai par rapporteur tel qu'un essai par luciférase ou Luminex.

6. Procédé selon l'une quelconque des revendications précédentes, dans lequel l'échantillon est du plasma ou du sérum.

7. Procédé selon l'une quelconque des revendications précédentes, comprenant les étapes de
(a) mise en contact de l'échantillon soupçonné de contenir un anticorps YB-1 avec YB-1 ou un fragment peptidique antigénique de cette dernière,
(b) détection de la liaison des anticorps à YB-1 ou au fragment peptidique antigénique de cette dernière.

8. Procédé selon la revendication 7, dans lequel le peptide est un peptide constitué de la séquence de SEQ ID NO : 2.

9. Procédé selon la revendication 7, dans lequel le peptide comprend 10 à 19, de préférence 12 à 19, résidus consécutifs de SEQ ID NO : 2, dans lequel le peptide a une longueur comprise entre 10 et 30, de préférence 12 et 24, résidus aminés.

10. Procédé selon les revendications 7 à 9, dans lequel le peptide est immobilisé sur une surface.

11. Procédé selon l'une quelconque des revendications 7 à 10, dans lequel la liaison est détectée à l'aide d'un anticorps secondaire contre la partie Fc de l'anticorps anti-YB-1.

12. Procédé selon la revendication 11, dans lequel l'anticorps anti-YB-1 est un anticorps IgG et l'anticorps secondaire est un anticorps anti-IgG.

13. Procédé selon les revendications 11 ou 12, dans lequel l'anticorps secondaire est marqué avec un marqueur détectable.

14. Utilisation in vitro de la protéine-1 de liaison à la boîte Y (YB-1) ou d'un fragment peptidique antigénique de cette dernière pour le diagnostic de la MA.

15. Utilisation selon la revendication 14, dans laquelle le peptide antigénique comprend au moins 10 à 19, de préférence 12 à 19, résidus consécutifs de SEQ ID NO : 2, et dans laquelle le peptide a une longueur comprise entre 10 et 30, de préférence 12 et 24, résidus aminés.
